# EUROPEAN PATENT APPLICATION

(11) **EP 4 684 769 A1**
(43) Date of publication of application: **28.01.2026**
(21) Application number: 24190393.9
(22) Date of filing: 23.07.2024
(51) Int. Cl.: A61F 9/007

(54) **SURGICAL TOOL**

(71) Applicant: Mann, Tristian Gater, Billingshurst RH14 9AA (GB)
(72) Inventor: Mann, Tristian Gater, Billingshurst RH14 9AA (GB)
(74) Representative: Schlich

(57) **Abstract**

A cutting tool, wherein the device comprises a first member having a first, distal portion and a second, proximal end portion, wherein a rotating member is mounted substantially at the distal end portion, wherein the rotating member is biased to rotate, and wherein one or more cutting members or blades may be mounted on the rotating member. The cutting tool may be for use in surgery, preferably eye surgery. Methods of use of the cutting tool and kits comprising the cutting tool are also described.

## Description

The present invention relates to surgical tools and devices for eye surgery. In particular the invention relates to tools suitable for performing corneal procedures, and most particularly capsulorhexis. The invention also encompasses methods of use of the tools of the invention, particularly with regard to corneal procedures and capsulorhexis of the human or animal eye.

Eye (ocular) surgery is a field with a long history but for which there is a need for improvements to yield greater effectiveness, efficiency, reliability, and safety of treatments. One condition of the eye that has affected humankind since prehistoric times is the development of cataracts of the eye ("cataracts").

The symptom of cataracts is the development of the cloudy area in the lens of the eye leading to a decrease in the acuity of vision. This loss of vision has been known and lamented since ancient times. Cataracts often develop slowly and can affect one or both eyes. Symptoms of cataracts may include blurred vision, perception of faded colours, the perception of halos around light sources, sensitivity to bright lights and impaired night vision. These primary symptoms may result in difficulty of inability to read, recognise faces accurately or drive a motor vehicle. Cataracts cause 51 % of all cases of blindness worldwide and 33% of visual impairment worldwide. Thus there is an ongoing and urgent need for more effective means of treatment of cataracts.

While the progression of cataracts may be ameliorated through diet and through control of exposure to the sun currently the only effective treatment for cataracts is by way of surgery. In particular, lens replacement surgery involves removal of the natural lens of the eye that has developed a cataract and replacement of the natural lens with an artificial lens. Such an artificial lens is referred to as an intraocular lens (IOL) implant.

A particular technique used during cataract surgery in order to access the lens is capsulorhexis (or capsulorrhexis) and more particularly the variant of this technique known as continuous curvilinear capsulorhexis (CCC). This surgical technique is used to remove the central anterior part if the capsule of the lens from the eye during cataract surgery. This is achieved by bringing shear and tensile forces to bear on the capsule of the lens. This can be achieved through the use of surgical forceps.

Alternatively, laser or electrothermic processes may be employed to make an incision or incisions in the capsule. Conventionally, capsulorhexis refers to the removal of the central part of the anterior lens capsule.

Capsulorhexis is important within the context of cataract surgery because in order to remove the lens by extracapsular techniques the capsule of the lens must be opened in order to gain access to the lens. Previous intracapsular cataract extractions involved the removal of the whole lens and capsule simultaneously. This was done to prevent any leftover lens material remaining at the operation site that might go on to cause an inflammatory response. However, more recent techniques allowing aspiration of practically all of the lens material have been developed yielding the advantage that the posterior capsule can be left intact. Leaving the posterior capsule intact advantageously provides a barrier between the front and back chambers of the eye and retains the vitreous humour jelly of the in the back chamber. A further benefit of retaining the barrier between the front and back chambers of the eye is that it provides a location for an IOL such that it can be held securely in place yet away from contact with other structures.

The original method of capsulorhexis utilised a small bent needle to make a series of incisions to form an effectively a continuous cut hole in the capsule around the anterior surface of the lens. The lens could then be removed through this aperture.

However this technique had the disadvantage of providing notched, angled, rough, or discontinuous edges which would cause areas of increased stress during removal of material. This increased stress could promote tears that would proceed outwards. Such a complication during cataract surgery is contraindicated. In contrast a continuous curvilinear capsulorhexis (CCC) will ideally have no notches or incisions at the edge and thus forces applied to the capsule during surgery are significantly better distributed and less likely to result in a tear. Thus the CCC technique yields a better result of treatment for a patient while also lessening the likelihood of intra-operative complications.

The current prevailing technique for CCC is to use a needle or similar to make a first incision in the capsule and then to extend or propagate the incision by means of tearing the tissue of the capsule in a controlled manner from outside the capsule around the anterior surface in order to provide a continuous, substantially circular, aperture in the capsule. The progress of the circumferential tear of the aperture can be guided or promoted using a needle or forceps or other surgical tool adapted for this purpose.

Thus continuous curvilinear capsulorhexis is a technique that is in common use because it has a low risk of initiating further outward tears in the capsule with the attendant benefits to the patient. However, such a technique is highly reliant on the skill of the surgeon performing the continuous curvilinear capsulorhexis.

Accordingly, there exists an ongoing need for surgical tools and techniques that will more reliably and more efficiently yield continuous curvilinear capsulorhexis (CCC).

In particular, cataract surgery is often carried out by a single surgeon on a "list" of patients. Typically a list of patients will comprise 16-20 patients. Also typically the surgeon will carry out the same surgical technique on each of the patients. This thus yields an economy of effort and use of the operating theatre, equipment, and employment of ancillary surgical staff.

Thus the number of patients who can be successfully treated during a single surgical session is related to the time and care that a surgeon must spend on each patient. If the time that a surgeon can spend on each patient is reduced by making an aspect of the surgical technique quicker and/or more reliable then additional patients can be added to the session list and, ultimately, a greater number of patients can be treated. Treatment of a greater number of patients within a specific time also yields greater efficiencies in terms of cost and resources to the organisation under whose aegis the cataract surgery is being carried out.

The steps of capsulorhexis after entering the eye through a paracentesis that is in current use are set out below:
1) Stabilization of the Eye:
   - Ensure the eye is physically stable, this may be done by injecting a viscoelastic substance to maintain the anterior chamber depth and protect the corneal endothelium.
2) Creation of Initial Tear:
   - A cystotome or capsulorhexis forceps are used to puncture the anterior lens capsule centrally or slightly off-centre. A cystotome is often fashioned from a 27- or 30-gauge needle.
3) Initiation of Tear:
   - With the cystotome or forceps, a small flap is created by lifting the edge of the initial puncture. Ensure this flap is well-defined to start the tearing process.
4) Formation of the Capsular Flap:
   - capsulorhexis forceps are used to grasp the flap and gently pull it in a circular motion. It is ideal at this stage to control the tear and guide it in a continuous circular path.
5) Guidance and Extension of Tear:
   - the tear around the anterior capsule is carefully extended, ideally maintaining a consistent radial distance from the centre. As noted above, ideally it is ensured that the tear does not run outwards, which could lead to complications.
6) Completion of Circular Tear:
   - Progress of the tear is guided until a circular opening, typically 5-6 mm in diameter, is achieved. Ideally, the edge of the capsulorhexis is smooth and continuous.
7) Verification:
   - the integrity of the capsulorhexis is verified to check that there are no radial tears or irregularities.

Throughout the capsulorhexis procedure, the anterior chamber's physical stability is normally maintained with viscoelastic as needed. A successful capsulorhexis is advantageous for the subsequent steps of cataract surgery, facilitating safe and effective removal of the lens nucleus and cortex, and providing a stable platform for IOL implantation.

Therefore there exists a need for surgical tools or techniques that yield a more rapid curvilinear continuous capsulorhexis (CCC). Furthermore, there exists an ongoing need for tools and technique that allow more reliable CCC. Furthermore, there exists an ongoing need for techniques and/or surgical tools that can be used by a less skilled practitioner while yielding comparable results to those delivered by skilled practitioners or surgeons using existing techniques.

There is also a need for such tools to be simple to manufacture and inexpensive to produce in large quantities.

The invention provides a cutting tool, wherein the device comprises a first member having a first, distal portion and a second, proximal end portion, wherein a rotating member is mounted substantially at the distal end portion, wherein the rotating member is biased to rotate, and wherein one or more cutting members or blades may be mounted on the rotating member. The proximal portion may comprise a handle. The handle may have a circular or elliptic cross section. Advantageously a handle with a circular or elliptic cross section may be better suited to the contours and grasp of a user's hand. The handle may have a geometric cross section (e.g. substantially square, rectangular, or hexagonal). Advantageously a handle with a geometric cross section can be conveniently placed in a stable position or otherwise stored. Additionally a geometric cross section can be accurately rotated with a reduced risk of slippage during movement.

Thus the invention provides a medical device. The medical device may be suitable for use in surgical procedures and may therefore be characterized as a surgical device.

The surgical device may be suitable for cutting or otherwise incising biological tissue. The tissue may be substantially any soft tissue of the human or animal body. However, the present tool of the invention is particularly suited to cutting the material of the capsule of the eye. Particularly the anterior capsule of the eye.

As noted above a key stage of eye surgery is capsulorhexis. Furthermore the advance in surgery of providing a continuous curvilinear capsulorhexis (CCC) is achieved through forming a continuously curved incision, cut, or tear in the capsule of the eye in order that the forces at the edge of the incision are evenly distributed thus further breaks radiating out from the cut are better prevented. Thus the rotating cutting means of the present invention is particularly well suited to providing an efficient and accurate capsulorhexis. Furthermore, the size of the capsulorhexis can be set by the act of locating of the cutting means on the rotating carriage or member prior to use or during manufacture.

The tool is particularly advantageous for eye surgery as the making of the cut, incision, or tear in the tissue is done from a position inside the capsule of the eye. This is in contrast to previous and conventional methods of continuous curvilinear capsulorhexis (CCC) where the tools to create the tear are located outside the capsule. In particular the known forceps are controlled by the user from outside the eye. Thus previously the cutting action brought to bear on the tissue of the eye originated from the outside of the eye or capsule of the eye. In contrast the present invention operates the cutting or tearing of the capsule is from inside the capsule. Furthermore, control of the cutting by the user activating of the release mechanism for the rotating part may originate from a signal or action of the user located outside the eye.

The tool may also be used in further eye surgeries. In some eye conditions, the innermost layer of the cornea called the "endothelium" is damaged. This causes the cornea to swell, affecting vision. Endothelial keratoplasty is a surgery to replace this layer of the cornea with healthy donor tissue. It is known as a partial transplant since only this inner layer of tissue is replaced. Such an operation is an advantageous way to restore vision when the inner cell layer of the cornea stops working properly from, e.g. Fuchs' dystrophy, bullous keratopathy, iridocorneal endothelial (ICE) syndrome, or other endothelial disorders. Advantageously endothelial keratoplasty selectively replaces only the diseased layer of the cornea, leaving healthy areas intact.

The device may also comprise a cutting member or spike mounted or located at the distal ends of the first member.

The terminal cutting member has the advantage of providing an initial incision through first the cornea then the proximal side of the capsule then the side of the capsule 180° opposite to the initial capsular incision this allows the tool to be inserted such that the rotating member can be located in the correct position in order to perform the circular cutting motion. The other advantage is that it creates the incision through the cornea which in conventional capsulorhexis is normally caried out by a separate bladed instrument This circular cutting motion is preferably capsulorhexis from inside the capsule as this is the key part of the invention.

The rotating member may be mounted near the tip of the first member the cutting member more proximally on the cutting member. The rotating member may be mounted near the tip of the first member the cutting member. The rotating member may be mounted more proximally on the cutting member. The rotating member may be mounted proximally of the cutting member or spike mounted at the distal end of the first member.

The plane of rotation of the rotating member may be substantially parallel to the longitudinal axis of the first member. Thus the axis of rotation of the rotating member may be substantially perpendicular to the longitudinal axis of the first member. This has the advantage a well-controlled plane of motion in relation to the first member and therefore an accurately placed and made incision resulting from its rotation. Thus the rotating member may be mounted on an axle that is mounted or integral with the first member, wherein the axle may be substantially perpendicular to the longitudinal axis of rotation of the first member.

The cutting members mounted on the rotating member terminal parts on both side or one may be mounted substantially perpendicular to the rotational plane of the member. This has the advantage of providing accurate and reproducible cutting or tearing. The cutting members may be located substantially at or near the end or terminus of the rotating member. This also has the advantage of the surgeon being able to more accurately perceive the limits of the circle being cut through the use of the tool. In this way the accuracy of the cut is better achieved.

The cutting or tearing member may be a blade. The blade may have a substantially straight, or curved cutting surface. The blade may have a cutting surface that is angled with respect to the rotational plane of the member. This has the advantage that the blade is carried at an angle through the material being cut. This yields a cleaner and more reliable cutting action. A further advantage of a blade that is not parallel to the rotational plane of the member is that the upmost point or edge of the blade can make the initial contact with the tissue of the eye or interior capsule and thus make a small or minimal incision that can be efficiently and/or accurately continued or propagated as the cutting member or tear is carried through the material being cut.

The cutting or tearing member may be a point or spike. This type of cutting member has the advantage of simplicity of manufacture and/or mounting on the rotating member. A further or alternative advantage is that the upmost tip of the point or spike can make the initial contact with the tissue of the eye or interior capsule and thus make a small or minimal hole or incision that can be efficiently and/or accurately continued or propagated as the cutting member is carried through the material being cut.

The cutting or tearing member may have a hook shape, or have a U- or V-shaped cutting surface. Such a cutting surface may make or propagates an incision by the material being cut being brought into contact with the base or pit of the hooked, or U- or V-shaped member. The arms of the V-shaped member may comprise bladed sections, preferably at the junction point of the arms, most preferably the bladed sections intersect or overlap at the junction point of the arms. The base of the hooked, or U- member may comprise a bladed portion. Without wishing to be bound by theory, advantageously shear forces are generated on the material being cut at the base or pit of the hooked, or U- or V-shaped member in order that a cut is made or propagated. This cutting action is advantageous as the shear forces add to the effect of the cutting force to yield greater reliability of cutting as the cutting member is carried through the material being cut.

The rotating member may be elongate. The rotating member being elongate has the advantage that it can be longitudinally aligned with respect to the first member such that its storage position before deployment (i.e. before beginning its rotational movement upon deployment) is substantially colinear and/or parallel with the first member and therefore can be introduced through a minimally sized aperture made in the tissue, preferably the cornea, most preferably the capsule of the eye.

The cutting surface at the distal end of the elongate member may directed axially and distally from the tool. The cutting surface at the distal end of the elongate member may be a point or spike directed axially and distally from the tool. The cross section of the distal cutting member viewed along an axis parallel to the longitudinal axis of the first member may be of sufficient area to obscure or shield the stored rotational member as it is passed through the aperture made by the distal cutting member, spike, or point.

The rotation of the cutting member may be biased to rotate, preferably the rotation is through an angle of approximately 180° or greater. The rotation of the cutting member may be biased to rotate through a pre-defined angle. The cutting member may rotate substantially through an angle selected from 180°, 360°, 540°, and 720°.

The cutting member may rotate substantially through an angle selected from 185°, 365°, 545°, and 720°.

The cutting member may rotate substantially through an angle selected from 190°, 370°, 350°, and 730°.

The cutting member may rotate substantially through an angle selected from 195°, 375°, 355°, and 735°.

The advantage of the rotation being greater than a half or full circle is that the cut is more reliable in that the user is better assured of the resulting cut being continuous. When one cutting member or blades is employed, without wishing to be bound by theory, rotation of a half circle (360°) is sufficient to yield a circular cut.

When two cutting members or blades are employed, without wishing to be bound by theory, rotation of a half circle (180°) may be sufficient to yield a circular cut.

The length of the cutting member may be selected in order to accommodate variation in the angular rotation of the member so that the cut is continuous.

The tool may cut a substantially circular area. The radius of the substantially circular area may be selected from 1mm, 1.1mm, 1.2mm, 1.3mm, 1.4mm, 1.5mm, 1.6mm, 1.7mm, 1.8mm, 1.9mm, 2.0mm, 2.1mm, 2.3mm, 2.4mm, 2.5mm, 2.6mm, 2.7mm, 2.8,mm, 2.9mm, 3.0mm, 3.1mm, 3.2mm, 3.3mm, 3.4mm, 3.5mm, 3.6mm, 3.7mm, 3.8mm, 3.9mm, 4.0mm, 4.1mm, 4.2mm, 4.3mm, 4.4mm, 4.5mm, 4.6mm, 4.7mm, 4.8mm, 4.9mm, 5.0mm, 5.1mm, 5.2mm, 5.3mm, 5.4mm, 5.5mm, 5.6mm, 5.7mm, 5.8mm, 5.9mm, 6.0mm, 6.1mm, 6.2mm, 6.3mm, 6.4mm, 6.5mm, 6.6mm, 6.7mm, 6.8mm, 6.9mm, 7.0mm, 7.1mm, 7.2mm, 7.3mm, 7.4mm, 7.5mm, 7.6mm, 7.7mm, 7.8mm, 7.9mm, 8.0mm, 8.1mm, 8.2mm, 8.3mm, 8.4mm, 8.5mm, 8.6mm, 8.7mm, 8.8mm, 8.9mm, and 9.0mm.

The device may comprise a detent or catch preventing rotation of the member. The position of the detent may be controlled via a linkage. The linkage may be attached to a member that slides collinearly with the handle portion 101. Thus advantageously the sliding portion of the handle may be conveniently used by the operator in order to release the detent when the cutting member is in the correct location. Release of the detent thus causes the biased rotation of the rotating member. Thus the cutting action may be controlled and released in the desired location.

The means of release of the detent may be a trigger or switch.

The device may comprise a stop or block preventing rotation of the member. The stop or block may be mobile to move from a position allowing rotary movement of the member to a position preventing movement of the member. Release of the detent may concomitantly move the blocking member to stop the rotating member after a defined amount or arc of movement.

The position of the stop or block may be controlled via a linkage. The linkage may be attached to a member that slides collinearly with the handle portion 101. Thus advantageously the sliding portion of the handle may be conveniently used by the operator in order to deploy the block when the cutting member has acted. Release of the detent thus ceases the biased rotation of the rotating member. Thus the cutting action may be advantageously controlled in the desired location.

Additionally the stop or block may be located so that the position of the rotating member is co-linear with the first member after the member travels through a defined amount of arc or movement. Accordingly, cooperating stops or blocks may be paired or located on the both the rotating member and the first member so that the rotation of the rotating member is halted when the member is co-linear with the first member after a defined amount of arc or movement of the rotating member. Thus the cross sectional area of the tool may be minimised after deployment or use of the cutting member to advantageously ease the withdrawal of the tool through the original aperture in the tissue.

Surgical tools or instruments (e.g. tools for eye surgery) and blades may be made from a variety of metals and alloys. The metal or alloy is chosen for specific properties such as strength, corrosion resistance, biocompatibility, and the ability to be sterilized.

These metals and their alloys may be thus selected based on the specific requirements of the surgical procedure, the type of instrument, and the desired properties such as hardness, flexibility, and corrosion resistance. The choice of material directly impacts the performance, durability, and safety of the surgical instruments. Thus the tools and/or, blades or cutting members may be made of a material selected from the following:
1) Stainless Steel
   - Types: 304, 316, 440C
   - Particular properties: High strength, excellent corrosion resistance, good edge retention.
   - Used, for example, in: Scalpels, scissors, forceps, needle holders.
2) Titanium
   - Types: Commercially pure titanium (CP), Titanium alloys (Ti-6AI-4V)
   - Particular properties: Lightweight, biocompatible, high strength-to-weight ratio, non-magnetic.
   - Used, for example, in: Microsurgical instruments, forceps, needle holders, scissors.
3) Platinum-Iridium
   - Particular properties: Extremely high corrosion resistance, excellent biocompatibility.
   - Used, for example, in: Fine precision instruments, microsurgical tools.
4) Cobalt-Chromium Alloys
   - Types: Vitallium
   - Particular properties: High wear resistance, excellent edge retention, good corrosion resistance.
   - Used, for example, in: Blades, cutting instruments.
5) Tungsten Carbide
   - Particular properties: Exceptional hardness, excellent edge retention, high wear resistance.
   - Used, for example, in: Cutting edges on scissors, needle holders, and other cutting instruments.
6) Molybdenum Alloys
   - Particular properties: High strength, good thermal conductivity, resistance to high temperatures.
   - Used, for example, in: Specialty surgical instruments.
7) Nitinol (Nickel-Titanium Alloy)
   - Particular properties: Shape memory, superelasticity, good biocompatibility.
   - Used, for example, in: Stents, guide wires, and other flexible instruments.
8) Silver
   - Particular properties: Antimicrobial properties, good conductivity.
   - Used, for example, in: Specialized instruments requiring antimicrobial properties.
9) Gold
   - Particular properties: Excellent biocompatibility, resistance to corrosion.
   - Used, for example, in: High-precision instruments, coatings on surgical blades.
10)Tantalum
   - Particular properties: High density, excellent biocompatibility, corrosion resistance.
   - Used, for example, in: Surgical clips, implants.

The invention provides a method of mass production of a device of the invention. Thus, advantageously, a tool of the invention may be produced efficiently enough or be inexpensive enough to be suitable for a single use (i.e. "disposable").

The invention also provides a method of use of a cutting tool, wherein the device comprises a first member having a first, distal portion and a second, proximal end portion, wherein a rotating member is mounted substantially at or near the distal end portion, wherein the rotating member is biased to rotate, and wherein one or more cutting members or blades are mounted on the rotating member.

The invention also provides for use of the device described herein in surgery. The surgery considered here may be eye surgery. The surgery considered here may be capsulorhexis, preferably continuous curvilinear capsulorhexis (CCC) or cutting of an endothelial graft for endothelial keratoplasty.

The invention also provides the use of the device of the invention in cutting a material. The material may be a biological tissue. Preferably the tissue is a soft tissue. Most preferably the tissue is the anterior capsule of the eye.

Accordingly, the invention also provides a method of cutting an aperture in a material in an otherwise inaccessible location. The inaccessible location may be in a volume that is substantially enclosed. Preferably the cutting member is admitted to the substantially enclosed volume through a small aperture that is of significantly smaller dimensions than the volume in which the cutting means operates.

The invention also provides a surgical system comprising a tool of the present invention. The apparatus may additionally comprise lighting equipment. The apparatus may additionally comprise a microscope.

The invention additionally provides a kit comprising a tool of the present invention.

The invention additionally provides a method of cutting material or tissue employing a device, apparatus, or kit of the invention.

The invention additionally provides a method of surgery employing a device, apparatus, or kit of the invention. The surgery may be carried out on a human or animal subject, preferably a human subject. The surgery may be eye surgery and is most preferably capsulorhexis of the eye or cutting of an endothelial graft for endothelial keratoplasty.

The invention also encompasses a use of a device, apparatus, or kit of the invention. Preferably the use of the device, apparatus or kit of the invention is in surgery. The surgery may be carried out on a human or animal subject, preferably a human subject. The surgery may be eye surgery and is most preferably capsulorhexis of the eye or cutting of an endothelial graft for endothelial keratoplasty.

### Examples and Drawings

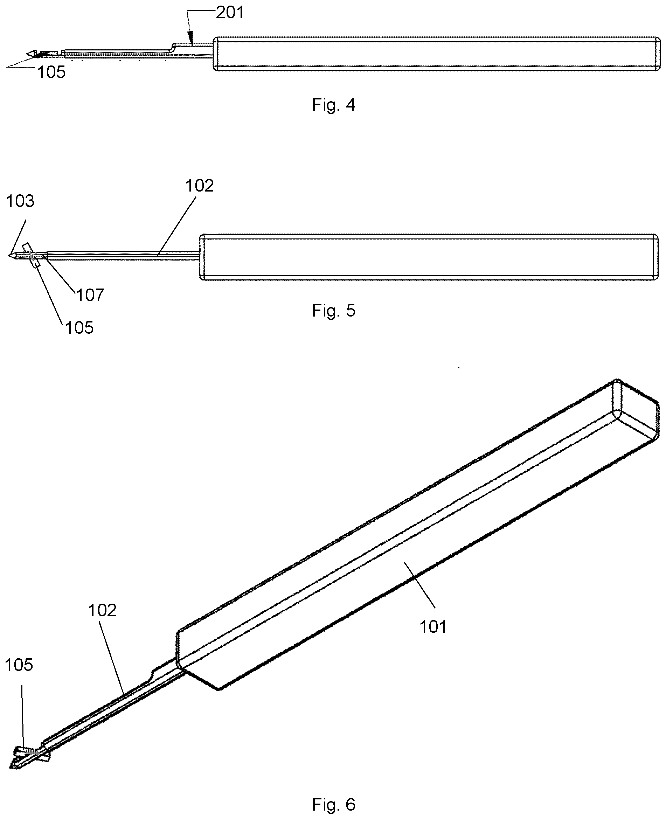

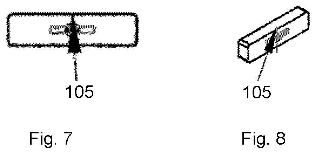

The invention will now be described with reference to the following drawings and examples in which:

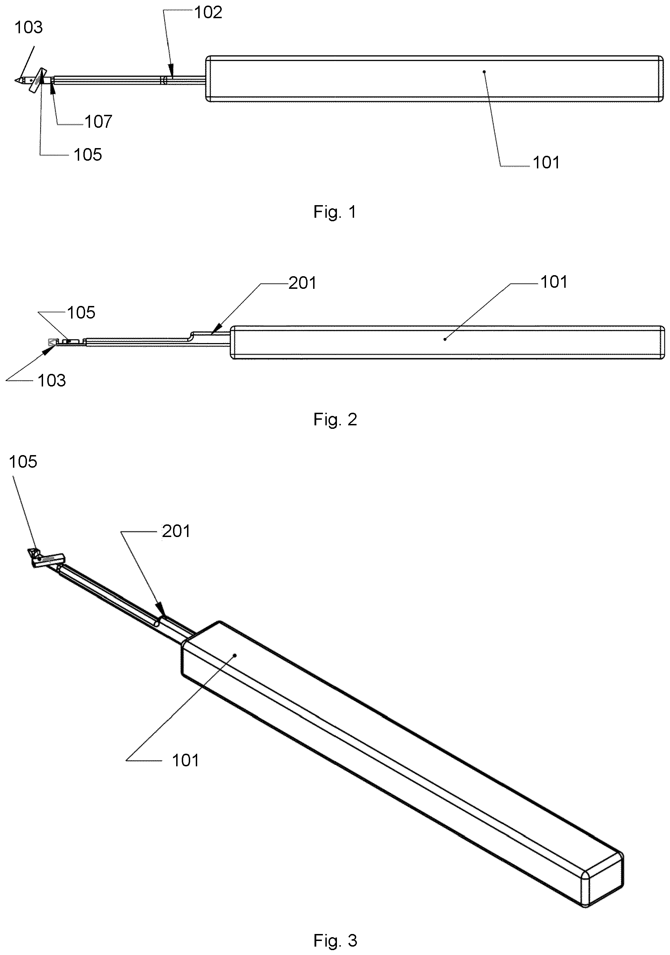

- Fig. 1: shows a top view of surgical tool having a handle 101 with a narrow elongate section 102 extending longitudinally therefrom. The end of the narrow elongate section 102 distal from the handle section 101 has a spike or sharpened point 103. Additionally, at the distal end of the longitudinal section 102 but located proximately to the sharpened section 103 is a rotating member 105 that rotates about an axis located perpendicular to the elongate section 102. The rotating member 105 has a bladed section mounted thereon. The blades (not shown) may be mounted so that the cutting edge is mounted substantially perpendicularly to the plane of rotation of the rotating member 105. The rotating member 105 is biased to rotate in a plane. The biasing means is preferably a spring. The biasing means may be a spring-loaded mechanism.. The biasing force is retained by a detent or catch 107. Upon release of the rotating member from retaining action of the detent the rotating member spins through substantially 180° or 360°. Thus the bladed sections contact the material to be cut and cut a circle preferably a perfect circle. Additionally, once the rotating member has rotated through 180° or 360° its movement is arrested by a further detent such that the rotating member is again is collinear with the longitudinally extended portion 102. The rotating member 105 is initially located collinearly with the longitudinal section 102 under the control of the detent. The blade section may be at one or both ends of the substantially cuboid rotating member. The axis of the rotating member is substantially centrally located. The rotating member 105 is shown partially rotated relative to its initial location.
- Fig.: 2 shows a side view of the apparatus of Figure 1. Figure 2 additional shows sliding part 201 which extends longitudinally from the handle part 101. Sliding part 201 causes release of the rotating member from the retaining detent so that the rotating member portion and the blades thereon may rotate to cut the desired tissue or material. The rotating member 105 is shown partially rotated relative to its initial location.
- Fig.: 3 shows a perspective view of the apparatus of Figures 1 and 2 showing the rotating carriage 107 partway through its motion, i.e. it is at an angle to the longitudinally extending section 102.
- Fig.: 4 shows the apparatus of Figure 3 a side view from an angle underneath the apparatus.
- Fig.: 5 shows a view from underneath the apparatus of Figures 3 and 4.
- Fig.: 6 shows a perspective view from oblique underneath of the apparatus of Figures 3 and 4 in the configuration shown in Figures 3 and 4.
- Fig.: 7 Shows a diagrammatic top view of rotating member 105.
- Fig.: 8 Shows a diagrammatic perspective view of rotating member 105.

### Examples

Capsulorhexis, an essential step in cataract surgery, involves creating a circular, continuous tear (e.g. CCC) in the anterior lens capsule to facilitate cataract removal and intraocular lens (IOL) implantation. The steps of capsulorhexis after entering the eye through a paracentesis are set out below:
1) Stabilization of the Eye:
   - Ensure the eye is physically stable, optionally this may be done by injecting a viscoelastic substance to maintain the anterior chamber depth and protect the corneal endothelium.
2) Creation of Initial Tear:
   - A cystotome or capsulorhexis forceps may be used to puncture the anterior lens capsule centrally or slightly off-centre. A cystotome is often fashioned from a 27- or 30-gauge needle. The tool of the invention may be inserted through this aperture.
   - Alternatively, the aperture may be made directly by the distal cutting member or point of the tool itself.
3) Insertion of the surgical tool of the invention
   - The surgical tool is inserted into the eye through the aperture made in the previous step. The tool is then located in position for the cutting members to be released to rotate and cut the tissue in a circular motion.
4) Operation of the surgical tool of the invention
   - With the surgical tool described herein, the detent is released and the rotating member moves under the operation of its biasing force to perform the circular cut in the eye tissue.
5) Completion of Circular Cut:
   - A circular opening, typically 5-6 mm in diameter, is produced. Ideally, the edge of the opening (capsulorhexis) is smooth and continuous.
6) Surgical tool of the invention is then be pulled out of the eye.
7) Verification:
   - the integrity of the capsulorhexis is verified to check that there are no radial tears or irregularities.

Throughout the capsulorhexis procedure, the anterior chamber's physical stability may be maintained with viscoelastic as needed. A successful capsulorhexis is advantageous for the subsequent steps of cataract surgery, facilitating safe and effective removal of the lens nucleus and cortex, and providing a stable platform for IOL implantation.

Considering the step of Operation of the surgical tool of the invention in more detail:
The surgical tool has a handle 101 with a narrow elongate section 102 extending longitudinally therefrom. The end of the narrow elongate section 102 distal from the handle section 101 has a spike or sharpened point 103. Additionally, at the distal end of the longitudinal section 102 but located proximately to the sharpened section 103 is a rotating member that rotates about an axle located perpendicular to the elongate section 102. The axis of the rotating member is substantially centrally located.

The rotating member 105 has a bladed section mounted thereon. The blade section may be at one or both ends of the substantially cuboid rotating member. The blade or blades are mounted so that the cutting edge is mounted substantially perpendicularly to the plane of rotation of the rotating member 105. The rotating member 105 is biased to rotate in a plane. The biasing means is preferably a spring. The biasing force is retained by a detent. The rotating member 105 is initially located collinearly with the longitudinal section 102 under the control of the detent 201 and its extension parallel to 102.

Upon release of the rotating member from retaining action of the detent the rotating member spins through substantially 180° or 360°. Thus the bladed sections contact the material to be cut and cut a circle preferably a perfect circle. Additionally, once the rotating member has rotated through its defined degree of movement (typically 180° or 360°) its movement is arrested by a further detent such that the rotating member is again is collinear with the longitudinally extended portion 102. shows a side view of the apparatus of Figure 1. Figure 2 additional shows sliding part 201 which extends longitudinally from the handle part 101. Sliding part 201 causes release of the rotating member from the retaining detent so that the rotating member portion and the blades thereon may rotate to cut the desired tissue or material.

## Claims

1. A cutting tool, wherein the device comprises a first member having a first, distal portion and a second, proximal end portion,
wherein a rotating member is mounted substantially at or near the distal end portion,
wherein the rotating member is biased to rotate, and
wherein one or more cutting members or blades are mounted on the rotating member.

2. A cutting tool according to claim 1, wherein the axis of rotation of the rotating member is substantially perpendicular to the longitudinal axis of rotation of the first member.

3. A cutting tool according to claim 1 or claim 2, wherein the tool is for use in surgery

4. A cutting tool according to any preceding claim, wherein the tool is for use in eye surgery

5. A cutting tool according to any preceding claim, wherein one or more cutting members or blades are mounted on the rotating member.

6. A cutting tool according to any preceding claim, wherein the rotating member is biased to rotate through a pre-determined angle.

7. A cutting tool according to any preceding claim, wherein the cutting members or blades are mounted substantially perpendicularly to the plane of rotation of the rotating member

8. A cutting tool according to any preceding claim, wherein the tool additionally comprises a cutting member or spike mounted at the distal end of the first member.

9. A cutting tool according to claim 7, wherein the rotating member is mounted proximally of the cutting member or spike mounted at the distal end of the first member.

10. A cutting tool according to any preceding claim, wherein the cutting member is located on or near the end or edge of the rotating member.

11. A cutting tool according to any preceding claim, wherein the distal part of the ? cutting member is located 2 to 3.5 mm from the axis of rotation of the rotating member.

12. A cutting tool according to any preceding claim, wherein the tool comprises a releasable detent or catch preventing the biased rotation of the rotating member

13. A kit comprising a tool of any preceding claim.

14. A method of cutting material employing a device or kit of any preceding claim, wherein the method comprises the steps of:
a) locating the tool so that the cutting members are in contact with the material to be cut; and
b) allowing the biased rotating member to move through a pre-determined path.

15. An apparatus comprising a tool of any of claims 1 to 12.
